# EUROPÄISCHE PATENTSCHRIFT

(11) **EP 2 910 203 B1**
(45) Veröffentlichungstag und Bekanntmachung des Hinweises auf die Patenterteilung: **01.11.2017**
(21) Anmeldenummer: 15153750.3
(22) Anmeldetag: 04.02.2015
(51) Int. Cl.: A61B 17/29, A61B 17/00

(54) **Medizinisches Instrument**
Medical instrument
Instrument médical

(30) Priorität: 19.02.2014 DE 102014102097
(43) Veröffentlichungstag der Anmeldung: 26.08.2015
(73) Patentinhaber: Karl Storz GmbH & Co. KG, 78532 Tuttlingen (DE)
(72) Erfinder: Kärcher, Daniel, 78532 Tuttlingen (DE); Stefan, Jochen, 78532 Tuttlingen (DE)

(56) Entgegenhaltungen:
- EP-A1- 2 532 315
- EP-B1- 2 316 358
- DE-A1-102011 085 512
- DE-A1-102012 007 649
- DE-U1- 9 418 094

## Beschreibung

Die Erfindung betrifft ein medizinisches Instrument mit einer proximalseitig angeordneten Handhabe, mit einem an der Handhabe angeordneten Schaft und mit einem am distalen Ende des Schaftes angeordneten Werkzeug.

Neben den einfachen medizinischen Instrumenten mit einer Funktionalität, wie z. B. das Öffnen und Schließen einer Schere als Werkzeug, sind im Rahmen von neueren Entwicklungen medizinische Instrumente bekannt, die mehrere verschiedene Funktionalitäten aufweisen. Beispielsweise ist aus dem deutschen Patent DE 197 80 579 B4 ein abwinkelbares, endoskopisches Instrument bekannt, das ein Werkzeug in Form eines zangenartigen Eingriffselementes zeigt und das eine zweite Funktionalität zeigt, die ein Schwenken des distalen Endes mit dem Werkzeug gegenüber dem restlichen endoskopischen Instrument ermöglicht. Zur Betätigung der beiden Funktionalitäten sind jeweils eigene, voneinander getrennte Betätigungsvorrichtungen vorgesehen, die am proximalen Ende des endoskopischen Instrumentes angeordnet sind. Bei dieser Anordnung der Betätigungselemente wurde eine ausreichend gute Handhabung und Bedieneffizienz erreicht. Durch die Vergrößerung der Funktionalitätenzahl wurde der endoskopische Einsatzbereich erweitert und dadurch der operative Aufwand bei der endoskopischen Operation, insbesondere durch eine Reduktion des Einführens und des Entnehmens aus dem Körper, verringert.

Diese Entwicklung wurde weitergeführt und mündete in einem medizinischen Instrument, das mehr als zwei Funktionalitäten zeigt. Dies ist ein Werkzeug, das geöffnet und geschlossen wird und das ein Rotieren des Werkzeuges sowie ein Verschwenken des distalen Endes mit dem Werkzeug gegenüber dem restlichen medizinischen Instrument ermöglicht. Dabei wird jede dieser Funktionalitäten durch eine eigene Betätigungsvorrichtung am proximalen Ende betrieben. Ein solches Instrument ist aus der deutschen Offenlegungsschrift DE 10 2012 007 649 A1 bekannt. Dieses Instrument erweist sich als sehr vielfältig in den Einsatzmöglichkeiten während einer endoskopischen Operation, jedoch bedarf es einer eingehende Schulung des Operateurs, damit eine verlässliche sichere Handhabung gerade unter schwierigen Operationsbedingungen gewährleistet ist.

Weiterhin ist dem europäischen Patent EP 2 316 358 B1 ein medizinisches Instrument zu entnehmen, das zwei Funktionalitäten aufweist, die über ein einziges gemeinsames Betätigungselement alternativ zueinander angesteuert werden können. Dabei ist das Betätigungselement in zwei Schaltstellungen bewegbar, wobei es in einer ersten Schaltstellung mit dem Schaft und in einer zweiten Schaltstellung mit dem Werkzeug bewegungsgekoppelt ist und damit entweder den Schaft rotieren kann oder das Werkzeug öffnen oder schließen kann. Dabei zeigt das Betätigungselement eine Schaltwelle, die zwei Kupplungsteile aufweist, die je nach Schaltstellung mit einem von zwei Gegenkupplungsteilen formschlüssig koppelt und dadurch die Möglichkeit schafft, durch Betätigung des Betätigungselementes über die Schaltwelle mit dem Kupplungsteil, das mit dem Gegenkupplungsteil formschlüssig koppelt, eine Bewegung auf das Werkzeug zum Öffnen oder Schließen bzw. auf den Schaft zum Rotieren zu übertragen. Dieses medizinische Instrument zeigt eine verbesserte Ergonomie, erweist sich aber in seiner Handhabungssicherheit als verbesserbar.

Die europäische Offenlegungsschrift EP 2 532 315 A1 lehrt ein medizinisches Instrument mit einem rotierbaren Betätigungselement, das in Längsrichtung verschiebbar ist. Je nach Position wird wirkt sich die Rotation des Betätigungselements auf einen ersten oder einen zweiten Abtrieb aus. Ein Ausführungsbeispiel dieser Erfindung umfasst ein Kupplungsteil mit elastisch verformbaren Elementen in Form von Blattfedern. Deren freie Enden greifen in Mitnahmeabschnitte des rotierbaren Betätigungselements ein bilden damit eine formschlüssige drehfeste Verbindung. Nachteilig dabei ist, dass die Torsionskräfte von den elastisch verformbaren Abschnitten des Kupplungsteils direkt übertragen werden, was den Einsatz von speziellen teuren Werkstoffen erfordert, welche gleichzeitig elastisch und scherfest sind.

Die deutsche Patentanmeldung DE 10 2011 085 512 A1 offenbart ebenfalls ein medizinisches Instrument mit einem längsverschiebbaren rotierbaren Betätigungselement. Um das Betätigungselement in seinen Funktionsstellungen zu fixieren, ist ein Rastelement im Griffteil vorgesehen, welches von einer Schraubenfeder in eine mit dem Betätigungselement verbundenen Ringnut gedrückt wird. Eine Rotationsbewegung überträgt dieses Rastelement nicht. Dieses gattungsgemäße medizinische Instrument wurde zur Bildung des Oberbegriffs herangezogen. Der vorliegenden Erfindung liegt die Aufgabe zugrunde, ein medizinisches Instrument zu schaffen, das eine verbesserte Ergonomie und Betätigungssicherheit bei mehr als einer Funktionalität zeigt.

Diese erfindungsgemäße Aufgabe wird durch ein medizinisches Instrument mit den Merkmalen des Anspruchs 1 gelöst. Vorteilhafte Weiterbildungen sind Gegenstand der abhängigen Ansprüche.

Das erfindungsgemäße medizinische Instrument zeigt eine proximalseitig angeordnete Handhabe, an der ein Schaft angeordnet ist, an dessen distalem Ende ein Werkzeug, insbesondere eine Schere, Zange oder ein HF-Koagulationswerkzeug angeordnet ist. Die Handhabe weist ein Betätigungselement auf, das eine Schaltwelle aufweist, die zusammen mit dem Betätigungselement zumindest in zwei Schaltstellungen bewegbar ist. In einer ersten Schaltstellung, die zur Aktivierung einer ersten Funktionalität vorgesehen ist, ist die Schaltwelle über ein erstes Kupplungsteil mit einem ersten Gegenkupplungsteil eines ersten Abtriebs zur Aktivierung der ersten Funktionalität mittels Formschluss bewegungsgekoppelt, während in einer zweiten Schaltstellung zur Aktivierung einer zweiten Funktionalität die Schaltwelle über ein zweites Kupplungsteil mit einem zweiten Gegenkupplungsteil eines zweiten Abtriebs zur Aktivierung der zweiten Funktionalität mittels Formschluss bewegungsgekoppelt ist. Dadurch lässt sich eine Bewegung des Betätigungselementes in der Handhabe je nach Schaltstellung über die Schaltwelle auf den ersten Abtrieb oder den zweiten Abtrieb übertragen und dadurch die jeweilige Funktionalität des Instrumentes betätigen. Dabei ist das erfindungsgemäße medizinische Instrument nicht nur auf zwei verschiedene Schaltstellungen zur Aktivierung selektiver Funktionalitäten beschränkt, sondern es können auch mehr als zwei unterschiedliche Funktionalitäten durch unterschiedliche Schaltstellungen aktiviert werden.

Erfindungsgemäß zeigen das erste und zweite Kupplungsteil jeweils wenigstens ein federndes Rastelement, die formschlüssig mit korrespondierenden Ausnehmungen an den zugeordneten Gegenkupplungsteile koppelbar ausgebildet sind. Vorzugsweise sind dabei mehrere federnde Rastelemente vorgesehen, die in mehrere korrespondierende Ausnehmungen eingreifen können. Alternativ dazu ist es erfindungsgemäß auch möglich, dass das erste oder zweite Gegenkupplungsteil jeweils ein oder mehrere federnde Rastelemente aufweisen, die in entsprechender Weise formschlüssig mit korrespondierenden Ausnehmungen am entsprechenden Kupplungsteil der Schaltwelle koppelbar sind. Durch diese erfindungsgemäße federnde Ausbildung der Rastelemente gelingt es die Handhabung und damit auch die Ergonomie des medizinischen Instrumentes zu verbessern, da das Einbringen der federnden Rastelemente in die korrespondierende Aufnahme zur Bildung des Formschlusses in der betreffenden Schaltstellung einfacher und insbesondere im Kraftverlauf angenehmer erfolgen kann. Darüber hinaus ist durch die federnde Ausbildung der Rastelemente ein besonders verlässlicher Formschluss gegeben, was sich sehr positiv auf die Sicherheit des medizinischen Instrumentes bei der Handhabung, insbesondere unter schwierigen Operationsbedingungen auswirkt. Dabei ist es gelungen, die Anzahl der notwendigen Betätigungselemente zur Betätigung mehrerer Funktionalitäten gering zu halten und damit die Ergonomie und Handhabbarkeit des medizinischen Instrumentes auf hohem Niveau zu erhalten. Es hat sich besonders bewährt, die federnden Rastelemente in einem Kupplungsteil oder Gegenkupplungsteil in einer Aufnahme so beweglich federnd anzuordnen, dass die federnden Rastelemente in der Aufnahme sich in einer Richtung auf die Mittelachse des Schaftes, also in radialer Richtung, zu oder weg bewegt werden können und dabei in formschlüssigem Eingriff in eine korrespondierende Ausnehmung oder aus dieser heraus bewegt werden können. Durch die bevorzugte Bewegungsführung der federnden Rastelemente durch die Aufnahme wird es möglich, sehr kompakt mehrere Paare von federnden Rastelementen und zugeordneten Aufnahmen kreisförmig um die Mittelachse des Schaftes in einem Kupplungsteil bzw. Gegenkupplungsteil anzuordnen, was zu einer sehr kompakten und sicheren Kopplung mittels Formschluss führt, die damit die Bewegung des Betätigungselementes sehr sicher und verlässlich auf den Abtrieb zur Betätigung der entsprechenden Funktionalität überträgt. Durch die federnde Ausbildung der Rastelemente, die ein flexibles federbewährtes Einpressen der Rastelemente in die korrespondierenden Aufnahmen bewirkt, ist es erfindungsgemäß möglich, einerseits im eingekoppelten Zustand das Rastelement möglichst tief, insbesondere bis auf den Grund der Ausnahme in diese einzuführen, und dadurch eine sehr sichere formschlüssige Kopplung zu erreichen und andererseits bei Bedarf entgegen der Federkraft das Rastelement in die Ausnehmung zurückzubewegen und dadurch eine Flexibilität und Sicherheit während des Schaltvorgangs zu schaffen, die der Stand der Technik mit starren Rastelementen und Ausnehmungen nicht zeigt.

Durch das Vorsehen eines Anschlags zur Begrenzung der insbesondere radialen Beweglichkeit der federnden Rastelemente gelingt es die Sicherheit des medizinischen Instrumentes bei Erhalt der vorteilhaften Ergonomie zu gewährleisten, da ein Herausgleiten und damit Trennen des beweglichen Rastelementes aus der Aufnahme und damit die Gefahr des Verlierens verhindert ist. Dabei wird der Anschlag insbesondere durch Ausbildung einer Verengung in der Aufnahme realisiert, die eine Begrenzung der verschieblichen Rastelemente bewirkt. Durch das Vorsehen von Federelementen zur Realisierung der federnden Rastelemente ist eine Anordnung geschaffen, die insbesondere durch Federkraft die federnden Rastelemente in Richtung des Anschlags durch Federkraft bewegt. Dabei ist im maximal ausgelenkten Zustand der Rastelemente ein sicheres Eingreifen in die zugeordnete, korrespondierende Aufnahme ermöglicht.

Neben der Möglichkeit den Anschlag durch ein zusätzliches Bauteil im Kupplungsteil oder Gegenkupplungsteil zu realisieren hat es sich besonders bewährt, den Anschlag und das Kupplungsteil oder Gegenkupplungsteil einstückig zu realisieren, was die Stabilität des Gesamtteils erhöht und damit die Sicherheit des erfindungsgemäßen medizinischen Instrumentes verbessert. Dabei hat es sich als vorteilhaft erwiesen, nicht nur einen Anschlag zur Begrenzung der Bewegung des federnden Rastelementes vorzusehen, sondern zwei, zwischen denen das Rastelement sich federbewährt frei bewegen kann. Dabei ist der federseitige Anschlag so gewählt, dass die Feder bevorzugt nur teilweise zusammengepresst ist, so dass eine Beschädigung der Feder verhindert werden kann. Neben dem Vorsehen einer Druckfeder ist alternativ auch das Vorsehen einer Zugfeder mit entsprechend angepassten Anschlägen oder auch eines Elastomer-Federelements möglich.

Durch die bevorzugte Ausbildung der Schaltwelle mit einer rotationssymmetrisch um den Umfang der Schaltwelle angeordneten Ausnehmungen und / oder federnden Rastelementen gelingt es die Materialbelastung der Schaltwelle gering zu halten und dadurch die Verlässlichkeit und Dauerhaftigkeit der Funktion des erfindungsgemäßen Instrumentes im besonderen Maße sicherzustellen. Besonders vorteilhaft hat sich das Vorsehen von drei oder vier federnden Rastelementen bzw. Ausnehmungen in jedem Kupplungsteil der Schaltwelle bewährt, die rotationssymmetrisch, also gleichmäßig um den Umfang der Schaltwelle bzw. dessen Kupplungsteil angeordnet ist. Durch diese besonders vorteilhafte Zahl an Kopplungselementen zum Formschluss an einem Kupplungsteil bzw. korrespondierend an dem Gegenkupplungsteil ist eine sehr effiziente optimierte Kraftverteilung bei reduziertem Fertigungsaufwand gegeben. Diese Anordnung erweist sich als überraschend sicher und in der Handhabung sehr ergonomisch.

Daneben hat es sich alternativ oder zusätzlich besonders bewährt, die Schaltwelle spiegelsymmetrisch so auszubilden, dass ihre beiden Enden mit den Kupplungsteilen wechselseitig zueinander spiegelsymmetrisch ausgebildet sind. Hierdurch lässt sich die Fertigungsqualität und damit die Sicherheit des medizinischen Instrumentes zusätzlich erhöhen, da die Anzahl der unterschiedlichen Bauteile reduziert ist und dadurch die Fertigung vereinfacht und dadurch das Risiko einer Fehlfunktion verringert und dadurch die Sicherheit des medizinischen Instrumentes erhöht wird.

Durch das vorteilhafte Vorsehen einer Verjüngung im Endbereich der Schaltwelle gelingt es auf besonders vorteilhafte Weise bei der Überführung des Betätigungselementes mit der Schaltwelle von einer Schaltstellung in die andere eine sichere Positionierung der Kupplungsteile in den Gegenkupplungsteilen zu erreichen, da mithilfe der durch die Verjüngung gebildeten Spitze eine vorteilhafte Ausrichtung, insbesondere in Verbindung mit einer entsprechend spitz zulaufenden Ausnehmung im Bereich der Gegenkupplung gegeben ist. Ein schädliches Verkanten der Schaltwelle im Bereich der Schaltstellungen kann damit verhindert werden. Bevorzugt wird eine solche Verjüngung an jedem Ende der Schaltwelle vorgesehen.

Durch das Vorsehen eines umlaufenden Rads oder eines Nockens als Teil des Betätigungselementes ist die Möglichkeit geschaffen, insbesondere von Hand sehr einfach und verlässlich und damit ergonomisch sicher, das Betätigungselement zu bewegen und zu betätigen. Dabei kann das Betätigungselement in unterschiedlicher Weise, beispielsweise durch Drehen aber auch durch axiales Verschieben betätigt werden, so dass zum einen die Möglichkeit geschaffen wird, mittels einer der Betätigungsarten die Schaltstellung und damit die gewünschte Funktionalität des erfindungsgemäßen medizinischen Instrumentes zu wählen und mit der anderen Betätigungsweise die Funktionalität des medizinischen Instrumentes in der gewünschten Weise zu aktivieren und damit zu betätigen.

Das Vorsehen eines umlaufenden Rades ermöglicht beispielsweise ein praktisch blindes Betätigen durch Drehen in beliebigen Positionen, ohne dass der Benutzer einen Blick auf das Betätigungselement werfen muss. Damit ist eine sehr einfache Handhabung geschaffen. Das Vorsehen eines einzigen Nockens ermöglicht ein zielgerichtetes Ergreifen des Nockens und ein sehr einfaches und differenziertes Betätigen in den unterschiedlichen Betätigungsweisen, was sich nach dem Auffinden des Nockens als sehr funktionell und ergonomisch erweist. Gerade das Vorsehen eines alternativen einzigen Nockens ermöglicht darüber hinaus auch das bevorzugte Koppeln des Betätigungselementes mit einem motorischen Antrieb, indem der Antrieb formschlüssig mit dem Nocken verbunden wird und dadurch die Bewegung des motorischen Antriebs auf das Betätigungselement verlässlich übertragen wird. Neben der Möglichkeit einer formschlüssigen Verbindung mithilfe des einen einzigen Nockens bestehen auch weitere Möglichkeiten mit mehreren Ausnehmungen und Erhebungen. Vorzugsweise ist diese formschlüssige Verbindung zwischen dem motorischen Antrieb und dem Betätigungselement lösbar ausgebildet.

Das Betätigungselement zur Bewegungsübertragung auf die Abtriebe ist bevorzugt jeweils um eine, insbesondere gemeinsame Drehachse drehbar, d. h. je nachdem in welcher Schaltstellung sich das Betätigungselement befindet, ist durch eine Drehung des Betätigungselementes immer ein einzelner bestimmter Abtrieb drehbar und damit eine bestimmte, diesem Abtrieb zugeordnete Funktionalität aktivierbar. Weiter bevorzugt ist das Betätigungselement parallel zu seiner Drehachse in seine Schaltstellungen axial verschiebbar, wodurch eine sehr einfache und verlässliche und damit ergonomische Bedienung gewährleistet ist.

Vorzugsweise kann das Betätigungselement neben den Schaltstellungen, in denen es über die Schaltwelle mit den Abtrieben bewegungskoppelt ist, auch in mindestens eine weitere Schaltstellung bewegbar sein, in der das Betätigungselement mit keinem der Abtriebe für eine Funktionalität bewegungsgekoppelt ist. Dadurch ist eine Neutralstellung des Betätigungselementes geschaffen, die gerade für das Überprüfen der Funktionalitäten bzw. die Wartung beziehungsweise das Auseinander- oder Zusammenbauen des erfindungsgemäßen medizinischen Instrumentes von besonderer Bedeutung ist. Dadurch gelingt es die dauerhafte Sicherheit des erfindungsgemäßen Instrumentes zu verbessern.

Als besonders vorteilhaft hat es sich erwiesen, eine oder mehrere, insbesondere alle Ausnehmungen mit einer Anlauffläche zu versehen, die mit abnehmendem Abstand zur Schaltstellung eine zunehmende Rückstellkraft des in die Ausnehmung eingreifenden Federelementes bewirkt. Dies sichert eine sehr gute und verlässliche Handhabung des erfindungsgemäßen medizinischen Instrumentes während des Schaltvorgangs. Durch diese Ausbildung wird zu Anfang des Überführens in eine Schaltstellung die Widerstandskraft, die dadurch entsteht, dass gegen die Federkraft das Rastelement beim Auftreffen auf die Schaltwelle im Bereich der korrespondierenden Ausnehmung verkürzt wird, gering ist und dadurch eine Positionierung sehr einfach und verlässlich gewährleistet werden kann, wohingegen mit zunehmenden Erreichen der Schaltposition der Widerstand durch die Ausbildung der Anlauffläche langsam vergrößert wird und dadurch ein formschlüssiges Einpressen der Rastelemente in die Ausnehmung und dadurch ein sehr verlässlicher Formschluss gewährleistet ist.

Darüber hinaus hat es sich besonders bewährt, ergänzend oder alternativ zu der Anlauffläche in der Ausnehmung, die Ausnehmung so auszubilden, dass im Bereich einer Schaltstellung eine Ablauffläche vorgesehen ist, durch die mit abnehmendem Abstand zu der betreffenden Schaltstellung eine abnehmende Rückstellkraft des Rastelementes vorgesehen ist. Hierdurch gelingt es eine Mulde in der Ausnehmung zu bilden, die eine Rastwirkung beim federnden Eingriff des Rastelementes in die muldenartige Ausnehmung bewirkt. Dadurch gelingt es eine haptische Rückmeldung des sicheren Erreichens der Schaltstellung zu erreichen, die die Handhabung und die Ergonomie des erfindungsgemäßen medizinischen Instrumentes verbessert. Die Abmessungen der Mulde werden dabei an die räumlichen Gegebenheiten, insbesondere die Größe der Rastelemente und das gewünschte Maß an haptischer Rückwirkung angepasst. Eine tiefe Mulde, die ein starkes Eindringen des Rastelementes in die Mulde ermöglicht und mit einem starken tiefen Gradienten verbunden ist, führt zu einer deutlichen haptischen Rückmeldung, während eine flache Mulde nur zu einer geringen haptischen Rückmeldung führt. Zusätzlich führt die Ablauffläche zu einer Eigenpositionierung des Rastelementes relativ zu der Schaltwelle mit den muldenförmigen Ausnehmungen, da durch die Federwirkung die Rastelemente in die Mulde gepresst werden und dadurch ein lateraler Schub durch die Ablaufkante erzeugt wird, der die Schaltwelle in die Schaltposition bewegt. Dies unterstützt die verbesserte Handhabung.

Eine besonders bevorzugte Ausbildung des erfindungsgemäßen medizinischen Instrumentes zeigt Ausnehmungen und / oder korrespondierende federnde Rastelemente, die einzeln oder teilweise oder alle konisch so ausgebildet sind, dass sie sich mit abnehmendem Abstand zu der Schaltstellung lateral verjüngen oder sich lateral vergrößern. Durch diese konisch, keilförmige Ausbildung kann sichergestellt werden, dass ein geringer seitlicher Versatz während des Schaltvorgangs, d. h. dem Übergang in die Schaltstellung und damit einer Annäherung an die Schaltstellung, aufgrund der Positionierwirkung der konischen Gestaltung der Ausnehmungen bzw. der Rastelemente zu einem seitlichen Verschieben, insbesondere in Form einer rotatorischen Neupositionierung, führt. Dadurch kann ein unerwünschtes Blockieren oder Behindern des Schaltvorgangs weitgehend ausgeschlossen werden, was die Sicherheit und die Handhabbarkeit und Ergonomie des vorliegenden erfindungsgemäßen medizinischen Instrumentes besonders verbessert.

Als besonders vorteilhaft hat sich dabei erwiesen, nicht nur einen Teil der Rastelemente derart konisch auszubilden, sondern alle Rastelemente eines Kupplungsteils bzw. Gegenkupplungsteils und zusätzlich die entsprechenden korrespondierenden Ausnehmungen mit der entsprechend korrespondierenden konischen Formgebung zu versehen, so dass ein besonders wirksamer Formschluss gegeben ist, der eine hohe Kraftübertragung ermöglicht. Es ist nicht notwendig, dass alle Rastelemente bzw. alle Ausnehmungen konisch ausgebildet sind, vielmehr kann dies auch nur teilweise der Fall sein aber auch eine Kombination aus konisch geformten und nicht konisch geformten korrespondierenden Elementen ist möglich und wird nach Bedarf und Fertigungsmöglichkeiten gewählt.

Ein besonders sicheres und gut handhabbares erfindungsgemäßes medizinisches Instrument zeigt eine Schaltwelle, die ausschließlich Ausnehmungen in ihren Kupplungsteilen aufweist, die insbesondere rotationssymmetrisch um den Umfang der Schaltwelle angeordnet sind und zu den Ausnehmungen korrespondierende federnde Rastelemente und / oder an den Gegenkupplungen, die mit dem jeweiligen Abtrieb zur Betätigung der Funktionalität des medizinischen Instrumentes verbunden sind. Durch diese Ausbildung gelingt es die Schaltwelle kompakt und leicht auszubilden, was die Betätigung mittels des Betätigungselementes vereinfacht und sicherer macht. Zudem erweist sich diese Schaltwelle als erfindungsgemäß konstruktiv wenig komplex. Die erfindungsgemäße Anordnung der korrespondierenden federnden Rastelemente in der Gegenkupplung, die nicht für den Schaltvorgang verschieblich bewegt wird, führt zu einer sehr robusten und verlässlichen Anordnung, die wenig anfällig für Defekte ist und einfach zu fertigen ist. Insbesondere ist die modulare Fertigung der einzelnen Komponenten, Gegenkupplung mit federnden Rastelementen und Abtrieb sowie die Fertigung der Schaltwelle mit Betätigungselement ermöglicht. Insgesamt führt diese bevorzugte Ausbildung der Erfindung zu einem sehr kompakten, verlässlichen und sicheren medizinischen Instrument, das sich durch besondere Ergonomie auszeichnet.

Es hat sich darüber hinaus als vorteilhaft erwiesen, das medizinische Instrument mit dem federnden Schaltgetriebe so auszubilden, dass bevorzugterweise paarweise als Alternativen entweder zum einen die Funktionalität Drehen des Schaftes bzw. des Werkzeuges und zum anderen Betätigen des Werkzeuges oder zum einen die Funktionalitäten Drehen des Schaftes bzw. Werkzeuges und zum anderen das Abwinkeln des Werkzeuges gegenüber der Längsachse des medizinischen Instruments bzw. Schaftes des medizinischen Instrumentes wählbar sind. Diese Kombinationen ermöglichen eine besonders vorteilhafte und effiziente und ergonomische Handhabung des medizinischen Instruments. Daneben hat es sich auch bewährt, das Abwinkeln gegenüber der Längsachse des medizinischen Instrumentes bzw. des Schaftes mit der alternativen Funktionalität der Werkzeugbetätigung zu kombinieren. Bei der Werkzeugbetätigung hat sich insbesondere das Öffnen und Schließen des Werkzeuges, z. B. in Form einer Zange oder Schere aber auch beispielsweise das Handhaben eines HF-Werkzeuges, beispielsweise durch Vergrößern der HF-Schlaufenelektrode oder Verkleinern dieser oder ein zusätzliches Auslenken des Werkzeuges, besonders bewährt.

Mit anderen Worten zeichnet sich das erfindungsgemäße medizinische Instrument mit mehreren Funktionalitäten dadurch aus, dass mittels eines Betätigungselementes zwischen diesen Funktionalitäten umgeschaltet werden kann, insbesondere durch das Verschieben eines Betätigungselementes, und dass in den verschiedenen Schaltpositionen unterschiedliche Kupplungs- und Gegenkupplungsteile formschlüssig ineinandergreifen, indem nicht starre Komponenten formschlüssig ineinandergreifen, sondern zumindest teilweise bewegliche Komponenten, die federbewehrt sind, den Formschluss zumindest partiell sicherstellen. Durch die flexiblen, positionsverändernden, formschließenden Teile ist das Einnehmen der Schaltstellung auf einfachere Weise ermöglicht, ohne dass die Formschlusswirkung in relevantem Umfang beeinträchtigt wäre. Damit erweist sich das erfindungsgemäße medizinische Instrument als ergonomisch besonders vorteilhaft ausgebildet. Es erweist sich zusätzlich auch als besonders sicher in der Handhabung.

Im Folgenden wird die Erfindung anhand eines Beispiels in den Figuren beschrieben. Die Erfindung ist nicht auf dieses Beispiel beschränkt.

Es zeigen:
- Fig. 1: ein beispielhaftes, erfindungsgemäßes medizinisches Instrument in einer Seitenansicht,
- Fig. 2: einen schematischen Längsschnitt durch einen Teil der Handhabe eines erfindungsgemäßen medizinischen Instrumentes,
- Fig. 3: einen schematischen Querschnitt durch einen Abschnitt der Handhabe entlang der Schnittlinie A - A gemäß Fig. 2 und
- Fig. 4: einen anderen Querschnitt durch einen Teil der Handhabe entlang der Schnittlinie B - B gemäß Fig. 2.

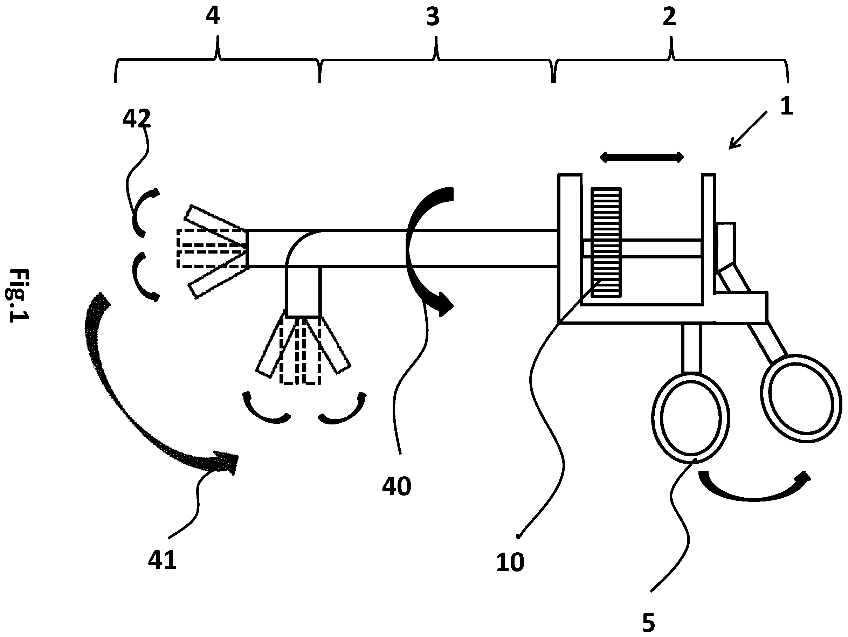

Das in Fig. 1 beispielhaft dargestellte endoskopische, medizinische Instrument 1 zeigt eine Handhabe 2, die am proximalen Ende des medizinischen Instrumentes 1 angeordnet ist. An die Handhabe 2 schließt sich distal der Schaft 3 an, an dessen distalem Ende wiederum das Werkzeug 4 angeordnet ist. Bei dem Werkzeug 4 handelt es sich um eine medizinische Schere oder Zange, die zwei Maulteile aufweist, die aufeinander zu- und voneinander wegbewegt werden können. Diese Bewegung 42 des Werkzeuges 4 ist durch den angedeuteten Pfeil 42 repräsentiert. Das distale Ende des Schaftes 3 mit dem Werkzeug 4 kann als eine weitere Funktionalität des medizinischen Instrumentes 1 abgewinkelt werden, wobei diese Abwinklungsbewegung 41 mit dem entsprechenden Pfeil 41 repräsentiert dargestellt ist. Die Abwinklung erfolgt dabei gegenüber dem Schaft 3, der die Hauptachse des medizinischen Instrumentes 1 bildet. Das medizinischen Instrumentes 1 zeigt als weitere Funktionalität die Möglichkeit, den Schaft 3 in eine Drehbewegung 40 zu versetzen, in der das Werkzeug 4 mit dem Schaft 3 um die Längsachse des Schaftes herum in Rotation versetzt wird. Diese Drehbewegung 40 ist durch den Drehpfeil 40 repräsentiert dargestellt.

Mithilfe der Handhabe 2, am proximalen Ende des medizinischen Instrumentes 1 können die unterschiedlichen Funktionalitäten angesteuert werden. Mithilfe des Griffes 5 wird das Werkzeug 4 geöffnet und geschlossen. Mithilfe des Betätigungselementes 10 wird die Funktionalität der Drehbewegung 40 sowie der Abwinklungsbewegung 41 gesteuert. Dabei erfolgt die Ansteuerung der beiden Bewegungen 40 und 41 alternativ zueinander, wobei dies abhängig von der Positionierung des Betätigungselementes 10 erfolgt und das Maß der Drehbewegung 40 bzw. der Abwinklungsbewegung 41 wird durch das Drehen des Betätigungselementes 10 bestimmt. Durch die Reduktion der Mittel zum Betätigen des medizinischen Instrumentes 1 ist eine wichtige Voraussetzung für eine effiziente, ergonomische Gestaltung eines medizinischen Instrumentes 1 mit einer Mehrzahl von Funktionalitäten 40, 41, 42 gegeben.

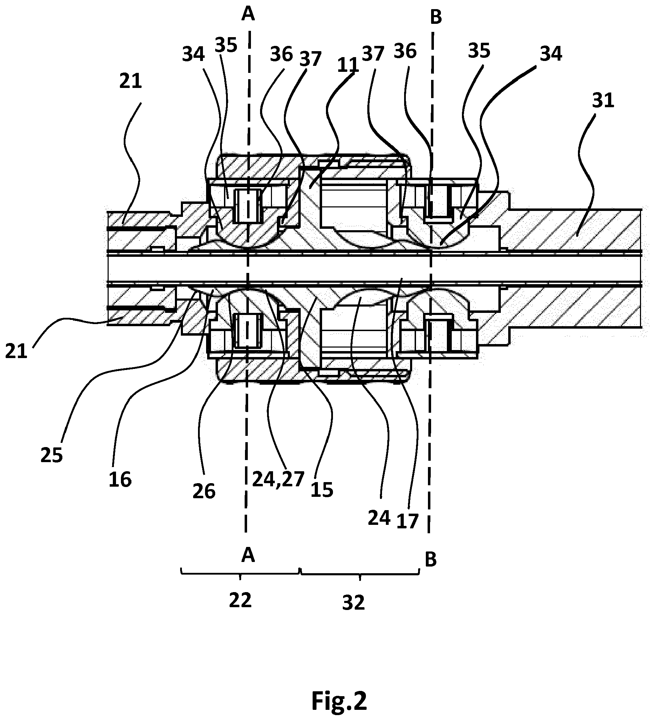

In Fig. 2 ist ein Längsschnitt durch einen Ausschnitt einer Handhabe 2 eines beispielhaften medizinischen Instrumentes 1 dargestellt.

Das Betätigungselement 10 zeigt einen radial abstehenden Nocken 11, der zur Bedienung und Betätigung einerseits hinsichtlich einer axialen Verschiebung und andererseits einer Drehbewegung um die Drehachse des Betätigungselementes 10 vorgesehen ist. Weiterhin zeigt das Betätigungselement 10 eine Schaltwelle 15, die zusammen mit dem Nocken 11 das Betätigungselement 10 bildet. Die Schaltwelle 15 erstreckt sich zu beiden Seiten des Nockens 11, der die Mitte der Schaltwelle 15 repräsentiert. Die Schaltwelle 15 zeigt einen symmetrischen Aufbau bezüglich des Nockens 11, d. h. das erste Ende 16 der Schaltwelle 15 zeigt denselben Aufbau wie das zweite Ende 17 der Schaltwelle 15, die sich zu beiden Seiten des Nockens 11 erstrecken. Dadurch ist ein konstruktiv einfacher Aufbau der Schaltwelle 15 gegeben, der die Fertigungssicherheit und dadurch die Qualität des medizinischen Instrumentes 1 erhöht.

Das Betätigungselement 10 kann axial zwischen zwei Schaltstellungen verschoben werden. In der in Fig. 2 dargestellten ersten Schaltstellung erstreckt sich das erste Ende 16 der Schaltwelle 15 mit dem daran angeordneten ersten Kupplungsteil 22 in ein erstes Gegenkupplungsteil 23, das mit einem ersten Abtrieb 21 zur Übertragung der Bewegung des ersten Kupplungsteils 22 auf den ersten Abtrieb 21 gekoppelt ist.

Das erste Kupplungsteil 22 der Schaltwelle 15 zeigt Ausnehmungen 24, in die federnde Rastelemente 34, die Teil des ersten Gegenkupplungsteils 23 sind, formschlüssig eingreifen. Dadurch ist ein Übertragen der Drehbewegung des Nockens 11 über die Schaltwelle 15 mit dem ersten Kupplungsteil 22 mit dessen Ausnehmungen 24 auf die federnden Rastelemente 34 des ersten Gegenkupplungsteils 23 und damit auf den ersten Abtrieb 21 ermöglicht. Dadurch lassen sich in der dargestellten ersten Schaltstellung mithilfe des Nockens 11 die Drehbewegung 40 des Schaftes 3 und damit des Werkzeuges 4 aktivieren.

Die federnden Rastelemente 34 sind dabei in dem ersten Gegenkupplungsteil 23, das das erste Ende 16 der Schaltwelle 15 in der ersten Schaltstellung ringförmig umschließt, in Aufnahmen 35 angeordnet. Die Aufnahmen 35 sind als radial verlaufende Ausnehmungen in der ersten Gegenkupplung 23 ausgebildet, die eine radiale Bewegungsführung der eingebrachten federnden Rastelemente 34 ermöglichen. In den Aufnahmen 35 ist zusätzlich ein Federelement 36 angeordnet, das eine Federkraft in radialer Richtung auf das federnde Rastelement 34 bewirkt. Sowohl am inneren Ende der Aufnahme 35 wie am äußeren radialen Ende der Aufnahme 35 ist jeweils ein Anschlag 37 vorgesehen, der die radiale Beweglichkeit des federnden Rastelementes 34 beschränkt. Dadurch ist ein Verbleib des Rastelementes 34 in der Aufnahme 35 und damit in der ersten Gegenkupplung 23 sichergestellt und damit die Funktionalität der federnden Rastelemente 34 gewährleistet.

Das federnde Rastelement 34 greift formschlüssig in der dargestellten ersten Schaltstellung in die korrespondierende Ausnehmung 24 des ersten Kupplungsteils 22 ein. Durch die Federkraft des Federelementes 36 wird das Rastelement 34 gegen den Boden der Ausnehmung 24 gepresst. Dabei ist die Auslenkung des federnden Rastelementes 34 im Rahmen der durch die Anschläge 37 bestimmten Bewegungsmöglichkeit abhängig von einem im Innenbereich des ersten Gegenkupplungsteils 23 angeordneten Gegenstand, der in der ersten Schaltstellung durch die Schaltwelle 15 mit dem ersten Kupplungsteil 22 gebildet ist.

Das erste Kupplungsteil 22 zeigt an seinem ersten Ende 16 eine konisch, sich verjüngende Spitze. Wird die Schaltwelle 15 axial in die erste Schaltstellung verschoben, so wird das konisch zulaufende erste Ende 16 der Schaltwelle 15 in den Bereich zwischen die voll durch die Federelemente 36 ausgelenkte Rastelemente 34 eingeführt. Die Rastelemente 34 treffen auf den Konus der Spitze und werden mit abnehmendem Abstand der Schaltwelle 15 zur ersten Schaltstellung gegen die Federkraft immer weniger ausgelenkt bis sie in die Ausnehmungen 24 gleiten und dann die abschließende Auslenkung in radialer Richtung in der ersten Schaltstellung eingenommen haben.

Durch die flexible, situationsabhängige Positionierung der federnden Rastelemente 34 ist eine sehr sichere und angenehme Handhabung des medizinischen Instrumentes 1 beim Einnehmen der Schaltstellung gegeben. Dabei wird durch den konischen Verlauf des ersten Endes 16 des ersten Kupplungsteils 22 der Schaltwelle 15 ein langsames Ansteigen der Widerstandskraft gegen das Einnehmen der ersten Schaltstellung erreicht, was die sichere und angenehme Handhabung bewirkt. Dies wird zudem dadurch erreicht, dass der Boden der Ausnehmung 24 einen Bereich aufweist, der eine Anlauffläche zeigt, die entsprechend dem konisch verlaufenden ersten Ende 16 der Schaltwelle 15 den Radius der Schaltwelle 15 erhöht und damit das anliegende Rastelement 34 gegen die Federkraft des Federelementes 36 zurückdrängt.

Durch das Vorsehen einer Ablauffläche 26 in der Ausnehmung 24 gelingt es ein Rasten des federnden Rastelementes 34 in der Ausnehmung 24 zu bewirken, sobald dieses in der ersten Schaltstellung gelangt ist. Dies wird dadurch erreicht, dass der Radius der Schaltwelle 15 mit zunehmendem Abstand vom ersten Ende 16 der Schaltwelle 15 abnimmt und dadurch beim Einbringen in die erste Schaltstellung mit abnehmendem Abstand zu dieser Schaltstellung eine abnehmende Rückstellkraft des federnden Rastelementes 34 gegeben ist, da dieses aufgrund des abnehmendem Radius stärker ausgelenkt werden kann. Diese Rastwirkung gibt dem Benutzer eine haptische Rückmeldung, dass die erste Schaltstellung erreicht ist. Zudem führt diese Ablauffläche 26 dazu, dass das Einnehmen dieser ersten Schaltstellung durch die Federkraft unterstützt wird und damit das Einnehmen dieser ersten Schaltstellung vereinfacht wird und zugleich das ungewollte Herausgleiten aus dieser ersten Schaltstellung aufgrund der steigenden Federkraft beim Herausbewegen verhindert werden kann. Dadurch ist eine sehr sichere und angenehme ergonomisch vorteilhafte Handhabung des erfindungsgemäßen Instruments 1 mit dem Schalten zwischen verschiedenen Schaltstellungen gegeben.

Das zweite Ende 17 mit dem zweiten Kupplungsteil 32 der Schaltwelle 15 zeigt den entsprechenden Aufbau wie das erste Ende 16 mit dem ersten Kupplungsteil 22. Entsprechendes gilt für den Aufbau des zweiten Gegenkupplungsteils 33, das dem ersten Gegenkupplungsteil 23 entspricht und mit dem zweiten Abtrieb 31 bewegungskoppelt. Es zeigt dieselbe Anordnung aus federnden Rastelementen 34, die in Aufnahmen 35 radial durch Federelemente 36 federbelastet beweglich ausgebildet sind. Ihre radiale Beweglichkeit ist dabei ebenso durch Anschläge 37 begrenzt. In Fig. 2 ist das zweite Gegenkupplungsteil 33 und das zweite Kupplungsteil 32 in einem nicht gekoppelten Zustand dargestellt. Dieser Zustand stellt die erste Schaltstellung dar. Durch axiales Verschieben des Betätigungselementes 10 mit der Schaltwelle 15 verlässt die Schaltwelle 15 die dargestellte erste Schaltstellung, der Formschluss zwischen dem ersten Kupplungsteil 22 und dem ersten Gegenkupplungsteil 23 mit ihren federnden Rastelementen 34 und den korrespondierenden Ausnehmungen 24 wird aufgelöst, das Betätigungselement 10 befindet sich in einer Zwischenstellung, in der die Schaltwelle 15 weder mit dem ersten Gegenkupplungsteil 23 noch mit dem zweiten Gegenkupplungsteil 33 formschlüssig und bewegungsgekoppelt ist und dadurch keine Bewegungskraft auf die beiden Abtriebe 21, 31 übertragen kann. Wird das Betätigungselement 10 weiter axial in Richtung zweites Gegenkupplungsteil 33 verschoben, so tritt es, wie zuvor entsprechend für das erste Gegenkupplungsteil 23 beschrieben, in Formschluss mit dem zweiten Gegenkupplungsteil 33 und ermöglicht dadurch eine Bewegungsübertragung von dem Betätigungselement 10 auf den zweiten Abtrieb 31 zur Aktivierung der zugeordneten Funktionalität.

Durch die erfindungsgemäße federnde Ausbildung der Rastelemente 34 zum formschlüssigen Eingriff in die zugeordneten, korrespondierenden Ausnehmungen 35 der Kupplungsteile 22, 32 bzw. der Gegenkupplungsteile 23, 33 ist einerseits eine sehr sichere und gute Kraftübertragung zur Aktivierung der entsprechenden Funktionalität des medizinischen Instrumentes 1 gewährleistet, da durch die Federkraft ein besonders guter Formschluss gewährleistet ist, der durch das Anliegen der Rastelemente 34 in den Ausnehmungen 24 geprägt ist. Zum anderen ist auch eine sehr angenehme und ergonomisch vorteilhafte Überführung des Betätigungselementes 10 in die verschiedenen Schaltstellungen durch die Federwirkung, insbesondere in Verbindung mit den verschiedenen Ausbildungen der Schaltwelle 15 mit den verschiedenen Anlaufflächen 25 und Ablaufflächen 26 sowie der konischen Ausbildung der Enden 16, 17 der Schaltwelle 15 gegeben. Dies führt insgesamt zu einem sehr sicheren und ergonomisch vorteilhaften Ausbilden des erfindungsgemäßen medizinischen Instrumentes 1 mit einer reduzierten Anzahl an Betätigungsvorrichtung 10 an der Handhabe 2 und einer größeren Anzahl an Funktionalitäten als die Anzahl der Betätigungvorrichtungen.

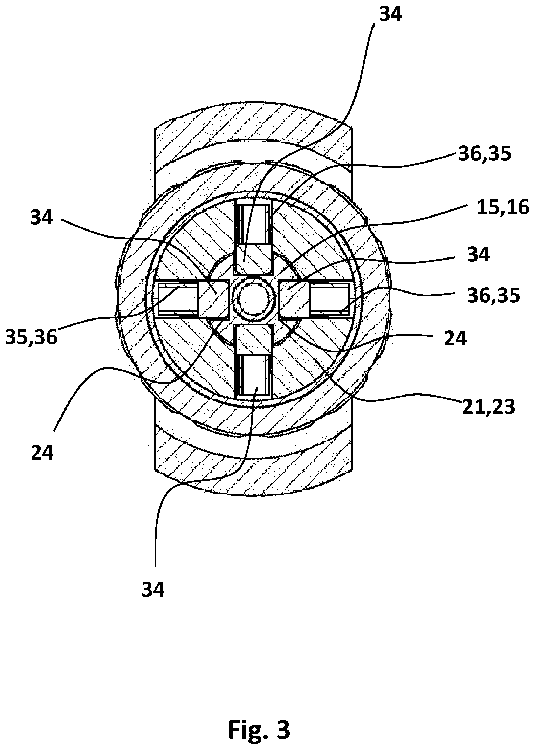

In Fig. 3 ist ein Querschnitt durch das erste Kupplungsteil 22 und das erste Gegenkupplungsteil 23 in einer ersten Schaltstellung entlang der Schnittlinie A - A entsprechend der Fig. 2 dargestellt.

Das erste Kupplungsteil 22, das am ersten Ende 16 der Schaltwelle 15 angeordnet ist, zeigt vier radial symmetrisch und damit gleichmäßig über den Umfang der Schaltwelle 15 angeordnete Ausnehmungen 24, die alle dieselbe Gestalt aufweisen. In diese vier Ausnehmungen 24 greifen formschlüssig vier federnde Rastelemente 34 ein, die Teil des ersten Gegenkupplungsteils 23 sind. Die federnden Rastelemente 34 sind in einem ringförmig ausgebildeten Gehäuse des ersten Gegenkupplungsteils 23 angeordnet, in dem für jedes Rastelement 34 eine Aufnahme 35 angeordnet ist, die sich in radialer Richtung erstreckt und an ihrem jeweiligen radialen Ende jeweils einen Anschlag 37 zeigt, der die radiale Beweglichkeit des federnden Rastelementes 34 beschränkt. Dadurch ist ein Herausgleiten oder Verlieren des Rastelementes 34 aus dem ersten Gegenkupplungsteil 23 verhindert. In den Aufnahmen 35 ist jeweils ein Federelement 36, das in Form einer Druckfeder realisiert ist, vorgesehen. Das Federelement 36 drückt mithilfe seiner Federkraft das Rastelement 34 radial nach innen in Richtung der Drehsymmetrieachse des ersten Gegenkupplungsteils 23. Die radiale Beweglichkeit ist, wie in Fig. 3 dargestellt, durch den Boden der Ausnehmungen 24 des ersten Kupplungsteils 22 begrenzt. Dort schlägt das federnde Rastelement 34 an und bewirkt dadurch eine sehr sichere und verlässliche, formschlüssige Kopplung, die eine gute Kraftübertragung und Bewegungsübertrag vom Betätigungselement 10 auf den ersten Abtrieb 21 gewährleistet.

Das erste Gegenkupplungsteil 23 zeigt dabei einen Aufbau aus zwei Ringen, die ineinander gefügt einen gemeinsamen Ring bilden. In den inneren Ring sind die Aufnahmen 35 als Ausnehmungen mit dem inneren Anschlag 37 eingebracht. Im Rahmen der Fertigung des Gegenkupplungsteils 23 wird zuerst in die Aufnahmen 35 des inneren Rings jeweils ein Rastelement 34 eingebracht, gefolgt von dem zugeordneten Federelement 36. Danach wird diese Einheit in den äußeren zweiten Ring, der geschlossen ausgebildet ist, eingebracht, so dass ein einziges gemeinsames, ringförmiges Gegenkupplungsteil 23 entsteht. Der äußere Ring bildet dadurch den zweiten radial außenliegenden Anschlag 37 für das Federelement 36, respektive für das Rastelement 34. Durch diese Ausbildung des Gegenkupplungsteils ist eine sehr sichere und einfache Fertigung für ein erfindungsgemäßes medizinisches Instrument 1 ermöglicht.

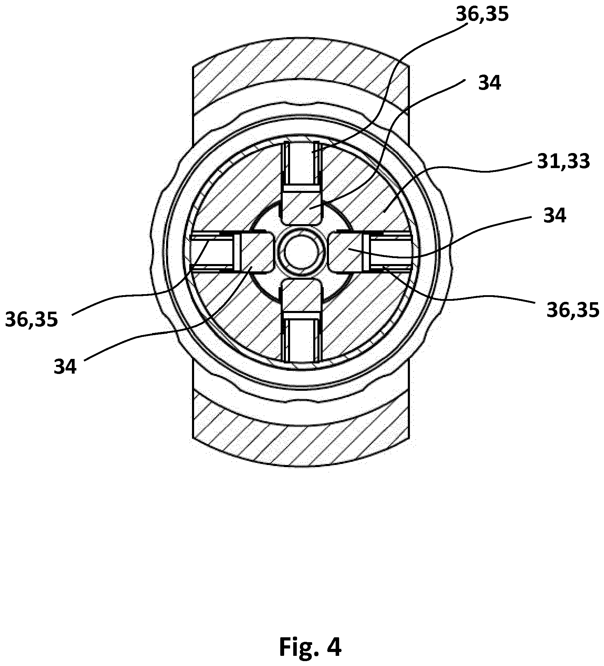

In Fig. 4 ist ein Querschnitt durch ein zweites Gegenkupplungsteil 33 während der ersten Schaltstellung aus der Fig. 2 entlang der Schnittlinie B - B dargestellt. Es zeigt einen entsprechenden Aufbau wie das erste Gegenkupplungsteil 23 der Fig. 2 bzw. der Fig. 3.

Im Gegensatz zu der Fig. 3 sind die federnden Rastelemente 34 weiter ausgelenkt, da sie nicht durch die Schaltwelle 15 in ihrer radialen Auslenkung begrenzt sind. In dieser dargestellten Situation kann mangels Formschluss zwischen Betätigungselement 10 mit der Schaltwelle 15 zu dem zweiten Gegenkupplungsteil 33 keine Kraftübertragung bzw. Bewegungsübertragung auf den zweiten Abtrieb 31 erfolgen. Damit wird deutlich, dass diese erste Schaltstellung, wie sie in Fig. 2 dargestellt ist, eine effiziente und sichere und ergonomisch vorteilhafte Bewegungskopplung zum ersten Abtrieb 21 und damit zur Aktivierung der zugeordneten Funktionalität, nämlich das Drehen des Schaftes 3, ermöglicht, während zugleich, wie in Fig. 4 dargestellt, eine Bewegungskopplung und mit dem zweiten Abtrieb 31 und damit eine Aktivierung der zugeordneten Funktionalität, nämlich dem Abwinkeln des Werkzeuges 4 gegenüber der Hauptachse des medizinischen Instrumentes 1 ausgeschlossen ist. Durch axiales Verschieben des Betätigungselementes 10 erfolgt auf die zuvor beschriebene vorteilhafte, ergonomisch ansprechende und sichere Weise das Einkoppeln des zweiten Kupplungsteils 32 in das zweite Gegenkupplungsteil 33 und damit das Erreichen der zweiten Schaltstellung, die einhergeht mit dem Auskuppeln des ersten Kupplungsteils 22 aus dem ersten Gegenkupplungsteil 23. Dadurch ist eine Bewegungsübertragung durch Formschluss der federnden Rastelemente 34 in die zugeordneten, korrespondierenden Ausnehmungen 24 des zweiten Kupplungsteils 32 ermöglicht und dadurch ein Übertragen der rotierenden Bewegung des Betätigungselementes 10 auf den zweiten Abtrieb 31 und damit eine Aktivierung der zweiten Funktionalität und damit ein Auslenken des Werkzeuges 4 gegenüber der Längsachse des medizinischen Instrumentes 1 bzw. des Schaftes 3 ermöglicht.

### Bezugszeichenliste

- 1: Medizinisches Instrument
- 2: Handhabe
- 3: Schaft
- 4: Werkzeug
- 5: Griff
- 10: Betätigungselement
- 11: Nocken
- 15: Schaltwelle
- 16: erstes Ende der Schaltwelle
- 17: zweites Ende der Schaltwelle
- 21: erster Abtrieb
- 22: erstes Kupplungsteil
- 23: erstes Gegenkupplungsteil
- 24: Ausnehmungen
- 25: Anlauffläche
- 26: Ablauffläche
- 27: Mulde
- 31: zweiter Abtrieb
- 32: zweites Kupplungsteil
- 33: zweites Gegenkupplungsteil
- 34: federnde Rastelemente
- 35: Aufnahme
- 36: Federelement
- 37: Anschlag
- 40: Drehbewegung des Schaftes
- 41: Abwinklungsbewegung des Werkzeuges
- 42: Bewegung des Werkzeuges beim Öffnen und Schließen

## Patentansprüche

1. Medizinisches Instrument (1) mit einer proximalseitig angeordneten Handhabe (2), mit einem an der Handhabe (2) angeordneten Schaft (3) und mit einem am distalen Ende des Schaftes (3) angeordneten Werkzeug (4),
wobei an der Handhabe (2) ein Betätigungselement (10) angeordnet ist, das in zumindest zwei Schaltstellungen bewegbar ist und eine Schaltwelle (15) aufweist,
wobei es in einer ersten Schaltstellung zur Aktivierung einer ersten Funktionalität (40) mit einem ersten Abtrieb (21) und in einer zweiten Schaltstellung zur Aktivierung einer zweiten Funktionalität (41) mit einem zweiten Abtrieb (31) des medizinischen Instrumentes (1) bewegungsgekoppelt ist,
wobei die Schaltwelle (15) ein erstes Kupplungsteil (22) aufweist, welches mit einem ersten Gegenkupplungsteil (23) des ersten Abtriebs (21) zur Aktivierung einer ersten Funktionalität (40) einen Formschluss bildet,
und wobei die Schaltwelle (15) ein zweites Kupplungsteil (32) aufweist, welches mit einen zweiten Gegenkupplungsteil (33) des zweiten Abtriebs (31) zur Aktivierung einer zweiten Funktionalität (41) einen Formschluss bildet,
**dadurch gekennzeichnet,**
**dass** das erste und zweite Kupplungsteil (22, 32) jeweils ein oder mehrere federnde Rastelemente (34) aufweisen, die formschlüssig mit korrespondierenden Ausnehmungen (24) am ersten und zweiten Gegenkupplungsteil (23, 33) koppelbar ausgebildet sind oder dass das erste und zweite Gegenkupplungsteil (23, 33) jeweils ein oder mehrere federnde Rastelemente (34) aufweisen, die formschlüssig mit korrespondierenden Ausnehmungen (24) am ersten und zweiten Kupplungsteil (22, 32) koppelbar ausgebildet sind,
**dass** die federnden Rastelemente (34) in Aufnahmen (35) in einem Kupplungsteil (22, 32) oder Gegenkupplungsteil (23, 33) in Radialrichtung beweglich federnd ausgebildet sind,
**dass** in den Aufnahmen (35) Federelemente (36) angeordnet sind, die eine Federkraft in radialer Richtung auf das federnde Rastelement (34) bewirken und
**dass** die Aufnahmen (35) für die federnden Rastelemente (34) einen Anschlag (37) zur Begrenzung der Beweglichkeit in Radialrichtung aufweisen.

2. Medizinisches Instrument nach Anspruch 1, **dadurch gekennzeichnet, dass** die Schaltwelle (15) rotationssymmetrisch und / oder beide Enden (16, 17) mit den Kupplungsteilen (22, 32) spiegelsymmetrisch zueinander ausgebildet sind.

3. Medizinisches Instrument nach einem der vorstehenden Ansprüche, **dadurch gekennzeichnet, dass** die Schaltwelle (15) zum einem oder zu mehreren Enden (16, 17) hin verjüngend ausgebildet ist.

4. Medizinisches Instrument nach einem der vorstehenden Ansprüche, **dadurch gekennzeichnet, dass** das Betätigungselement (10) ein umlaufendes Rad oder einen Nocken (11) zur Betätigung aufweist.

5. Medizinisches Instrument nach einem der vorstehenden Ansprüche, **dadurch gekennzeichnet, dass** das Betätigungselement (10) formschlüssig mit einem motorgetriebenen Antrieb verbunden ist.

6. Medizinisches Instrument einem der vorstehenden Ansprüche, **dadurch gekennzeichnet, dass** das Betätigungselement (10) zur Bewegungsübertragung auf die Abtriebe (21, 31) jeweils um eine gemeinsame Drehachse drehbar ist und in Richtung der Drehachse in seine Schaltstellungen axial verschiebbar ist

7. Medizinisches Instrument nach einem der vorstehenden Ansprüche, **dadurch gekennzeichnet, dass** das Betätigungselement (10) zur Bewegungsübertragung zwischen den Schaltstellungen zur Aktivierung verschiedene Funktionalitäten (40, 41) eine oder mehrere Zwischenstellungen aufweist, die keine Bewegungskopplung zu einem der Abtriebe (21, 31) für eine Funktionalität (40, 41, 42) aufweisen.

8. Medizinisches Instrument nach einem der vorstehenden Ansprüche, **dadurch gekennzeichnet, dass** eine oder mehrere Ausnehmungen (24) eine Anlauffläche (25) aufweisen, die mit abnehmenden Abstand zur Schaltstellung eine zunehmende Rückstellkraft des federnden Rastelementes (34) bewirkt.

9. Medizinisches Instrument nach einem der vorstehenden Ansprüche, **dadurch gekennzeichnet, dass** eine oder mehrere Ausnehmungen (24) eine Ablauffläche (26) aufweisen, die im Bereich einer Schaltstellung mit abnehmenden Abstand zu dieser Schaltstellung eine abnehmende Rückstellkraft des federnden Rastelementes (34) und damit ein Rasten bewirkt.

10. Medizinisches Instrument nach einem der Ansprüche 1 bis 3, **dadurch gekennzeichnet, dass** Ausnehmungen und / oder die korrespondierenden federnden Rastelemente (34) sich mit abnehmenden Abstand zu der Schaltstellung lateral verjüngen und / oder sich lateral vergrößern.

11. Medizinisches Instrument nach einem der vorstehenden Ansprüche, **dadurch gekennzeichnet, dass** 3 oder 4 korrespondierende federnde Rastelemente (34) und Ausnehmungen (24) in oder an den Kupplungsteilen (22, 32) oder Gegenkupplungsteilen (23, 33) ausgebildet sind.

12. Medizinisches Instrument nach einem der vorstehenden Ansprüche, **dadurch gekennzeichnet, dass** korrespondierenden Ausnehmungen (24) in den Kupplungsteilen (22, 32) der Schaltwelle (15) angeordnet sind und die korrespondierenden federnden Rastelemente (34) in oder an den Gegenkupplungsteilen (23, 33)angeordnet sind.

13. Medizinisches Instrument nach einem der vorstehenden Ansprüche, **dadurch gekennzeichnet, dass** die geschalteten Abtriebe (21, 31) die Werkzeugbetätigung insbesondere durch Öffnen oder Schließen, das Abwinkeln gegenüber Längsachse des medizinischen Instrumentes und / oder das Drehen um die Längsachse des medizinischen Instrumentes bewirken.

## Claims

1. Medical instrument (1) with a handle (2) arranged at the proximal end, with a shank (3) arranged on the handle (2), and with a tool (4) arranged at the distal end of the shank (3),
wherein an actuation element (10), arranged on the handle (2), is movable to at least two switch positions and has a selector shaft (15),
wherein, in a first switch position for the activation of a first functionality (40), it is movably coupled to a first output (21) and, in a second switch position for the activation of a second functionality (41), it is movably coupled to a second output (31) of the medical instrument (1),
wherein the selector shaft (15) has a first coupling part (22) which establishes a form fit with a first mating coupling part (23) of the first output (21) for the activation of a first functionality (40),
and wherein the selector shaft (15) has a second coupling part (32) which establishes a form fit with a second mating coupling part (33) of the second output (31) for the activation of a second functionality (41),
**characterized in that**
the first and second coupling part (22, 32) each have one or more resilient locking elements (34) which are designed to be able to couple with a form fit to corresponding recesses (24) on the first and second mating coupling part (23, 33), or **in that** the first and second mating coupling part (23, 33) each have one or more resilient locking elements (34) which are designed to be able to couple with a form fit to corresponding recesses (24) on the first and second coupling part (22, 32),
**in that** the resilient locking elements (34) in seats (35) in a coupling part (22, 32) or mating coupling part (23, 33) are designed to be movable resiliently in the radial direction,
**in that** spring elements (36), which apply a radial spring force to the resilient locking element (34), are arranged in the seats (35) and
**in that** the seats (35) for the resilient locking elements (34) have an abutment (37) for limiting the mobility in the radial direction.

2. Medical instrument according to Claim 1, **characterized in that** the selector shaft (15) is rotationally symmetrical and / or both ends (16, 17) with the coupling parts (22, 32) are designed in mirror symmetry to each other.

3. Medical instrument according to either of the preceding claims, **characterized in that** the selector shaft (15) is designed tapering toward the one or more ends (16, 17).

4. Medical instrument according one of the preceding claims, **characterized in that** the actuation element (10) has a rotating wheel or a cam (11) for the actuation.

5. Medical instrument according to one of the preceding claims, **characterized in that** the actuation element (10) is connected with a form fit to a motor-driven drive.

6. Medical instrument according to one of the preceding claims, **characterized in that** the actuation element (10) for the movement transmission to the outputs (21, 31) is in each case rotatable about a common rotation axis and is axially movable to its switch positions in the direction of the rotation axis.

7. Medical instrument according to one of the preceding claims, **characterized in that** the actuation element (10) for the movement transmission between the switch positions for the activation of different functionalities (40, 41) has one or more intermediate positions, which have no movement coupling to one of the outputs (21, 31) for a functionality (40, 41, 42).

8. Medical instrument according to one of the preceding claims, **characterized in that** one or more recesses (24) have a run-on surface (25) which, with a decreasing distance from the switch position, effects an increasing restoring force of the resilient locking element (34).

9. Medical instrument according to one of the preceding claims, **characterized in that** one or more recesses (24) have a run-off surface (26) which, in the area of a switch position, with a decreasing distance from this switch position, effects a decreasing restoring force of the resilient locking element (34) and, therefore, a locking action.

10. Medical instrument according to one of Claims 1 to 3, **characterized in that** recesses and / or the corresponding resilient locking elements (34) taper laterally and / or widen laterally with a decreasing distance from the switch position.

11. Medical instrument according to one of the preceding claims, **characterized in that** 3 or 4 corresponding resilient locking elements (34) and recesses (24) are formed in or on the coupling parts (22, 32) or mating coupling parts (23, 33).

12. Medical instrument according to one of the preceding claims, **characterized in that** corresponding recesses (24) are arranged in the coupling parts (22, 32) of the selector shaft (15), and the corresponding resilient locking elements (34) are arranged in or on the mating coupling parts (23, 33).

13. Medical instrument according to one of the preceding claims, **characterized in that** the switched outputs (21, 31) effect the tool actuation in particular by opening or closing, the pivoting relative to the longitudinal axis of the medical instrument and / or the rotation about the longitudinal axis of the medical instrument.

## Revendications

1. Instrument médical (1) avec une poignée (2) disposée du côté proximal, avec une tige (3) disposée au niveau de la poignée (2) et avec un outil (4) disposé au niveau de l'extrémité distale de la tige (3) ;
selon lequel un élément d'actionnement (10) est disposé au niveau de la poignée (2), lequel peut être déplacé dans tout au moins deux positions de commutation et lequel présente un arbre de commande (15) ;
selon lequel ledit élément d'actionnement est couplé en mouvement avec une première prise de force (21) dans une première position de commutation, en vue de l'activation d'une première fonctionnalité (40), et est couplé en mouvement avec une deuxième prise de force (31) de l'instrument médical (1) dans une deuxième position de commutation, en vue de l'activation d'une deuxième fonctionnalité (41) ;
selon lequel l'arbre de commande (15) présente une première pièce de couplage (22), laquelle forme une complémentarité de forme avec une première pièce de couplage femelle (23) de la première prise de force (21) en vue de l'activation d'une première fonctionnalité (40) ; et
selon lequel l'arbre de commande (15) présente une deuxième pièce de couplage (32), laquelle forme une complémentarité de forme avec une deuxième pièce de couplage femelle (33) de la deuxième prise de force (31) en vue de l'activation d'une deuxième fonctionnalité (41) ;
**caractérisé en ce que**,
la première et la deuxième pièce de couplage (22, 32) présentent respectivement un ou plusieurs éléments élastiques d'enclenchement par encliquetage (34), lesquels sont conçus de manière à pouvoir être couplés par complémentarité de forme avec des gorges (24) correspondantes au niveau de la première et de la deuxième pièce de couplage femelle (23, 33), ou **en ce que** la première et la deuxième pièce de couplage femelle (23, 33) présentent respectivement un ou plusieurs éléments élastiques d'enclenchement par encliquetage (34), lesquels sont conçus de manière à pouvoir être couplés par complémentarité de forme avec des gorges (24) correspondantes au niveau de la première et de la deuxième pièce de couplage (22, 32) ; et
**en ce que** les éléments élastiques d'enclenchement par encliquetage (34) sont conçus de manière élastique et mobile dans la direction radiale dans des logements (35) dans une pièce de couplage (22, 32) ou dans une pièce de couplage femelle (23, 33) ; et
**en ce que** dans les logements (35) sont disposés des éléments formant ressorts (36) qui actionnent une force élastique dans la direction radiale sur l'élément élastique d'enclenchement par encliquetage (34) ; et
**en ce que** les logements (35) pour les éléments élastiques d'enclenchement par encliquetage (34) présentent une butée (37) en vue de la délimitation de la capacité de déplacement dans la direction radiale.

2. Instrument médical selon la revendication 1, **caractérisé en ce que** l'arbre de commande (15) est conçu à symétrie de révolution avec les pièces de couplage et/ou **en ce que** les deux extrémités (16, 17) sont conçues en symétrie miroir, l'une par rapport à l'autre, avec les pièces de couplage (22, 32).

3. Instrument médical selon l'une des revendications qui précèdent, **caractérisé en ce que** l'arbre de commande (15) est conçu en se rétrécissant en direction de l'une ou de plusieurs extrémités (16, 17).

4. Instrument médical selon l'une des revendications qui précèdent, **caractérisé en ce que** l'élément d'actionnement (10) présente, en vue de l'actionnement, une roue en rotation ou une came (11).

5. Instrument médical selon l'une des revendications qui précèdent, **caractérisé en ce que** l'élément d'actionnement (10) est raccordé par complémentarité de forme à un entraînement commandé par un moteur.

6. Instrument médical selon l'une des revendications qui précèdent, **caractérisé en ce que** l'élément d'actionnement (10), en vue de la transmission du mouvement sur les prises de force (21, 31), est capable de rotation, respectivement autour d'un axe de rotation commun, et est capable de coulisser de manière axiale dans ses positions de commutation, dans la direction de l'axe de rotation.

7. Instrument médical selon l'une des revendications qui précèdent, **caractérisé en ce que** l'élément d'actionnement (10), en vue de la transmission du mouvement entre les positions de commutation, présente une ou plusieurs positions intermédiaires en vue de l'activation de diverses fonctionnalités (40, 41), lesquelles positions intermédiaires ne présentent, pour une fonctionnalité (40, 41, 42), aucun couplage en mouvement avec l'une des prises de force (21, 31).

8. Instrument médical selon l'une des revendications qui précèdent, **caractérisé en ce qu'**une ou plusieurs gorges (24) présentent une surface de butée (25), laquelle actionne une force de rappel croissante de l'élément élastique d'enclenchement par encliquetage (34) au fur et à mesure que la distance décroît par rapport à la position de commutation.

9. Instrument médical selon l'une des revendications qui précèdent, **caractérisé en ce qu'**une ou plusieurs gorges (24) présentent une surface de dégagement (26), laquelle actionne une force de rappel décroissante de l'élément élastique d'enclenchement par encliquetage (34) dans la zone d'une position de commutation, au fur et à mesure que la distance décroît par rapport à cette position de commutation et laquelle actionne par conséquent un cran.

10. Instrument médical selon l'une des revendications 1 à 3, **caractérisé en ce que** des gorges et/ou les éléments élastiques d'enclenchement par encliquetage (34) correspondants se rétrécissent latéralement et/ou s'agrandissent latéralement au fur et à mesure que la distance décroît par rapport à la position de commutation.

11. Instrument médical selon l'une des revendications qui précèdent, **caractérisé en ce que** 3 ou 4 éléments élastiques d'enclenchement par encliquetage (34) correspondants et gorges (24) sont conçus dans des pièces de couplage ou des pièces de couplage femelle ou au niveau des pièces de couplage (22, 32) ou des pièces de couplage femelle (23, 33).

12. Instrument médical selon l'une des revendications qui précèdent, **caractérisé en ce que** des gorges (24) correspondantes sont disposées dans les pièces de couplage (22, 32) de l'arbre de commande (15) et les éléments élastiques d'enclenchement par encliquetage (34) correspondants sont disposés dans des pièces de couplage femelle (23, 33) ou au niveau de ces dernières.

13. Instrument médical selon l'une des revendications qui précèdent, **caractérisé en ce que** les prises de force (21, 31) mises en commutation provoquent l'actionnement de l'outil, en particulier par l'ouverture ou par la fermeture, la courbure par rapport à l'axe longitudinal de l'instrument médical et/ou la rotation autour de l'axe longitudinal de l'instrument médical.
